(19) Europäisches Patentamt
European Patent Office
Office européen des brevets

(11) Publication number: 0 158 446 B1

(12) EUROPEAN PATENT SPECIFICATION

(45) Date of publication of patent specification: 31.08.88

(51) Int. Cl.$^4$: **C 08 G 65/30**, C 08 G 65/00, C 07 C 43/12

(21) Application number: 85301723.4

(22) Date of filing: 13.03.85

# (54) Process for decontaminating perfluoropolyethers.

(30) Priority: 15.03.84 IT 2006184

(43) Date of publication of application: 16.10.85 Bulletin 85/42

(45) Publication of the grant of the patent: 31.08.88 Bulletin 88/35

(84) Designated Contracting States: BE DE FR GB NL

(56) References cited:
EP-A-0 089 820
EP-A-0 106 317
DE-A-2 109 758
US-A-3 665 041
US-A-3 704 214
US-A-3 715 378

(73) Proprietor: AUSIMONT S.p.A.
31, Foro Buonaparte
I-20121 Milano (IT)

(72) Inventor: Caporiccio, Gerardo
13, via E. Filiberto
Milan (IT)
Inventor: Strepparola, Ezio
1/A, V. le Partigiano
Treviglio (Bergamo) (IT)

(74) Representative: Whalley, Kevin et al
MARKS & CLERK 57/60 Lincoln's Inn Fields
London WC2A 3LS (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 158 446 B1

Courier Press, Leamington Spa, England.

**Description**

This invention relates to a process for decontaminating perfluoropolyethers, i.e. perfluoropolyethereal oils, and more particularly to a process for removing from perfluoropolyethers, having a minimum vapour tension, organic polluting substances containing —OH groups and/or

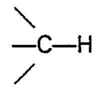

bonds.

There are known perfluoropolyethers having the structure represented by the following general formula:

$$R_f—(CF_2CFXO)_m(CF_2O)_n—R_f' \qquad (I)$$

wherein: X is F or $—CF_3$; $R_f$ and $R_f'$, which may be the same as or different from each other, are $—CF_3$, $—C_2F_5$, or $—C_3F_7$: n is zero or an integer and m is an integer such that m/n may be from 0.5 to 30, while the sum (m+n) is from 3 to 200; furthermore, when n is zero and X is $—CF_3$, then $R_f$ and $R_f'$ are preferably $—C_2F_5$ and $—C_3F_7$.

These products have high chemical, physical and thermal stabilities and exhibit marked lubricating properties due to which they are employable as operative fluids at a minimum vapour tension, for vacuum pumps of the mechanical or diffusion type.

Due to their chemical inertia they are suitable for being utilized in particular in the vacuum field when it is necessary to remove or to pump aggressive gases such as, for example, halogens, haloid acids, anhydrides of Lewis acids or Lewis acids themselves. During pumping some aggressive gases could come into contact with other compounds and react with such compounds, thus giving rise to other harmful compounds. For example, boron, aluminium and silicon halides could come into contact with moisture and hydrolize, forming compounds which pollute the operative fluids consisting of the perfluoropolyethers of formula (I).

Also, the aggressive gases could cause corrosive attack on the pump walls and the corrosion products may remain in the pump to contaminate the oil.

In conclusion, the perfluoroethereal oils of formula (I), which are very resistant by themselves, may progressively become charged with increasing amounts of insoluble and polluting products in suspension. As a consequence, these suspensions may prove harmful for the pump operation.

Other causes of pollution are contamination with organic or inorganic liquids, which may be carried into the perfluorinated oil either because they are sucked from the ambient atmosphere in which the vacuum has been created or because they were already present in the pump before it was supplied and filled with the perfluoropolyether of formula (I). In such case if the polluting products are only organic liquids, the reactivity thereof with respect to possible aggressive compounds usually pumped by means of perfluorinated liquids gives rise to heterogeneous mixtures having stability and resistance characteristics lower than those of the perfluoropolyethers of formula (I).

The perfluoropolyethers of general formula (I) can be used as lubricants in various fields, such as the mechanical and the electronic fields, to lubricate metal surfaces or surfaces of resinous materials, in reciprocal revolving or sliding motion, or as lubricants for magnetic recording means (tapes or discs).

The perfluoropolyethers are often distributed on the surface to be lubricated in the form of a solution in a suitable volatile solvent such as trichlorotrifluoroethane, in which case the application can be carried out by spraying or by immersion; during the treatment, accidental pollution may be caused by the perfluoropolyether solution which is to be recovered for economical reasons. This involves a purification by filtration and evaporation of the solvent. Or the perfluoropolyethers may come into contact, during their use as lubricants, with other liquids such as water, mineral oils, silicones, phosphoric esters, organic esters in general or other organic solvents. When, after a utilization period, it is necessary, for maintenance or repair operations, to clean the apparatus, the perfluoropolyether lubricant needs to be recovered, such recovery usually consisting of extraction with solvent, filtration, and evaporation of the solvent.

The processes generally known for the purification of perfluoropolyethereal oils from hydrogenated organic substances, from water, or from suspended metallic or inorganic solid residues, are based on physical separations such as filtration, centrifugation, extraction with solvent, and distillation. Drying can be operated by suitable dehydrating agents, which are brought into contact, for a prolonged period of time, with the perfluoropolyethereal oil, or by evaporation under vacuum or in a nitrogen stream under hot conditions.

The processes described hereinabove do not prove to be effect enough to satisfactorily remove the last traces of organic substances contained in the perfluoropolyethereal oils. As a consequence the perfluoropolyethers do not exhibit, after such treatments, the same properties of chemical resistance, stability to heat and to oxidation they originally possessed, due to such even very low contents of hydrogenated substances. For example, a perfluoropolyethereal oil resists an oxygen impact test, with oxygen being conveyed under a pressure of

$$1.621\times10^7—1.824\times10^7 \text{ Pa } (160—180 \text{ atm.})$$

and at a starting temperature of 60°C, while when

it is polluted by 0.1% by weight of hydrogenated substances, its resistance decreases to a level corresponding to a fourth or a fifth of the original one. The test is conducted by placing a substance sample into a high-pressure resisting container, charged with oxygen at $1.013\times10^5$ Pa (1 atm.) and brought to a test temperature of, for example, 60°C. The oxygen pressure is then suddenly increased, bringing it to a predetermined value of, e.g., $1.621\times10^7$ Pa (160 atm.) and then checking, in a short time, the temperature in the container. If such temperature is higher than that which would be caused by the adiabatic compression of the gas in the container, that means that an immediate oxidation reaction (combustion) has taken place.

It is therefore very important, when operating in the presence of oxygen, to have in the apparatus running with perfluoropolyethereal oil, charges of such oil free from hydrogenated impurities.

Furthermore, in the nuclear field it is often required that not more than 2—3 ppm of groups

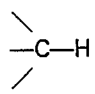

and not more than 1—2 ppm of groups —OH be present in order to avoid interactions of such groups with $UF_6$, an oxidizing and fluorinating substance which, by reaction at a temperature from 20° to 130°C, gives rise to uranyl fluoride $UOF_2$ or to uranium tetrafluoride $UF_4$, these being solid products with a high abrasive power which can damage moving parts in the separation apparatuses, such as for example supporting bearings of ultracentrifuges. These requirements are met by the perfluoropolyethereal oils prepared by synthesis starting from hexafluoropropene, by photo-oxidation and the subsequent process steps described in US 3,442,942, 3,896,167, 3,630,928, 3,699,145, 3,704,214 and 3,715,378 relating to the synthesis, in US 3,665,041 relating to neutralization, and in US 4,178,465 relating to the purification and fluorination with $ClF_3$.

Such requirements or specification cannot be complied with in perfluoropolyethereal oils which were polluted when they were used with organic or inorganic substances and which were successively purified by means of the physical methods of the art mentioned hereinbefore. Another example of high-purity requirements for the perfluoropolyethereal oils is encountered in the field of the manufacture of silicon and aluminium wafers by etching plasma processes, in which, in proper working chambers, in reactive ambient vapours, under reduced pressure of the order of 2.66 Pa ($2.10^{-2}$ Torr), the wafers are subjected to etching treatments by bombardment of fluorinated ions, or to the action of electric discharge on products such as $CCl_4$, $BF_3$, $BCl_3$, $PF_3$, $PCl_3$, $Cl_2$, $O_2$. Furthermore, in the plasma chamber, substances such as $AlCl_3$ and $SiF_4$ are generated; these products, having characteristics of Lewis acids, can be transported into the vacuum pump, and in the case that the operative oil should contain

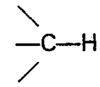

bonds and —OH groups, hydrolysis reactions of the Lewis acids and conversion of the hydrogenated organic substances may occur, with formation of insoluble solid residues which cause the wearing of the mechanical elements in motion.

The present invention provides a process for removing from perfluoropolyethers organic polluting substances containing —OH groups and/or

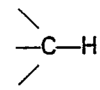

bonds, characterised by treating the perfluoropolyether in the liquid state, at a temperature from 150° to 200°C, with a gaseous reagent selected from oxygen, chlorine and fluorine, and in the presence of ultraviolet radiation thus obtaining the conversion of the polluting substances into volatile degradation products, which are successively removed by degassing the perfluoropolyether.

By means of the process according to the present invention it is possible to remove from perfluoropolyethers having a minimum vapour tension, also traces of impurities consisting of organic compounds containing bonds other than C—O, C—F, C—C, reducing such impurities to levels comparable with those which are typical of the perfluoropolyethers obtained from the above mentioned synthesis processes, i.e. to such contents as to be at the sensitivity limits of the analytical methods, for example to contents of groups

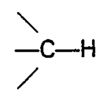

of 2—3 ppm and below and to contents of groups —OH of 1—2 ppm and below, by subjecting the perfluoropolyethereal oils containing the said organic impurities to a reaction with oxygen, or chlorine or fluorine, as agents capable of forming radicals which interact with

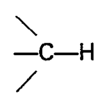

bonds and also with the —OH groups, or of inducing the formation of radicals in the said hydrogenated organic impurities.

Such treatment is accomplished by feeding the perfluoropolyether preheated to a temperature

from 150° to 200°C, in the liquid state, into a reaction chamber where it comes into contact with one of the gaseous reagents indicated hereinabove. To obtain a more effective action of such reagents, the reaction chamber is irradiated with ultra-violet rays preferably coming from a Hg vapour lamp and having a wavelength preferably from 240 to 600 nm.

The reaction with the organic impurities containing

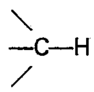

bonds and/or —OH groups causes the formation of volatile degradation products such as $CO_2$, HF, HCl, and carbon halides, which are successively removed from the perfluoropolyethereal oil by degassing under vacuum. The perfluoropolyether is inert under the said reaction conditions. After treatment, the product purity is checked by analyses of the type:

Fourier Transform (FT)—NMR.
Fourier Transformer (FT)—I.R.

or by means of the oxygen impact strength test, with oxygen being conveyed under a pressure of

$1.418\times10^7$—$1.824\times10^7$ Pa (140—180 atm.)

at a starting temperature of 60°C according to the test already described.

The process according to the invention is particularly useful when it is carried out downstream of conventional physical purification processes, such as filtration, centrifugation, and extraction with solvents, in order to remove the considerable amounts of hydrogenated and/or solid impurities from the perfluoropolyethereal oil. In fact, by means of such physical processes it is not possible to exhaustively remove the last trace of polluting compounds, which, however, may be removed by the process according to the invention.

The invention will be further described with reference to the following illustrative Examples.

Example 1

The process was conducted in a glass reactor having a capacity of 1.5 l, a length of 30 cm and an inside diameter of 8 cm, equipped with a heating band thermo-regulated at 150°C and, at the head, with a quartz sheath having an outside diameter of 6 cm, in which an ultraviolet-ray lamp type Hanau (Hanau is a Registered Trade mark) TQ81, 150 W power was immersed, with a pipe for the inflow of the perfluoropolyethereal oil, the end of such pipe being equipped with an orifice of 1 mm diameter, with a dipping pipe at the reactor bottom for feeding a reagent in the gas phase, with a vertical riser having a 2-cm diameter and a 20-cm length, connected to the upper end of a trap cooled by means of $CO_2$ and connected to a vacuum pump capable of maintaining a pressure of $2.66\times10^4$ Pa (200 Torr). Into the reactor, equipped with a discharge at the bottom connected with a surge tank maintained at $1.33\times10^2$ Pa (1 Torr), there were introduced, through the capillary orifice, first 100 g of perfluoropolyethereal oil preheated at 175°C, then gaseous fluorine at a rate of 10 Nml/min. The perfluoropolyether of formula (I), with X=$CF_3$, had a viscosity of $2.5\times10^{-4}m^2/s$ (250 c.St.) at 20°C and indicated, on FT—I.R. analysis, a content of

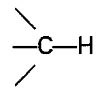

bonds of about 100 ppm and of —OH groups of about 50 ppm. The perfluoropolyethereal oil was further fed at a rate of 200 g/hour, while an equal amount thereof was discharged from the bottom discharge pipe. The discharged liquid was made to flow into the surge tank maintained at $1.33\times10^2$ Pa (1 Torr), in which degassing of the product was completed. The perfluoropolyether so obtained, subjected to FT—IR analysis, was found to contain not more than 2 ppm of

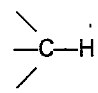

bonds and less than 1 ppm of —OH groups.

Example 2

The reactor of example 1 was utilized and was operated continuously, charging first 1000 g of perfluoropolyethereal oil preheated at 175°C. Gaseous chlorine was introduced at a rate of 20 N · ml/min. and irradiation with ultraviolet light was effected. The treated perfluoropolyethereal oil of formula (I), where X=$CF_3$, exhibited a viscosity of $2.5\times10^{-4}m^2/s$ (250 c.St.) at 20°C and on FT—IR analysis was found to contain about 100 ppm of

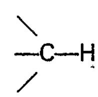

bonds and 50 ppm of —OH groups. 200 g/hour of oil to be treated were continuously fed and an equal amount of treated product was discharged, which was conveyed into the surge tank under a $1.33\times10^2$ Pa (1 Torr) vacuum. The product, analyzed at the surge tank outlet, was found to contain about 2 ppm of

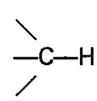

bonds and an equal amount of —OH groups.

Example 3

The same reactor of example 1 was utilized and the same procedure with the same ultraviolet

lamp was followed. 200 g/hour of a perfluoropolyethereal oil of formula (I), wherein X=F, having a viscosity of $2.5\times10^{-4} m^2/s$ (250 c.St.) at 20°C and containing, as indicated by FT—IR analysis, about 150 ppm of

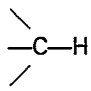

bonds and 80 ppm of —OH groups, were continuously introduced. 20 N · ml/min. of oxygen were introduced into the reactor, maintained at a pressure of $2.66\times10^4$ Pa (200 Torr) and at a temperature of 175°C. After treatment with oxygen, the perfluoropolyethereal oil was conveyed to the surge tank under a $1.33\times10^2$ Pa (1 Torr) vacuum for degassing and then it was discharged. The product so treated was found to contain, on FT—IR analysis, 1 ppm of—OH groups and, on FT—NMR analysis, 2 ppm of

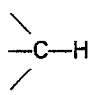

bonds.

**Claims**

1. A process for removing from perfluoropolyethers organic polluting substances containing —OH groups and/or

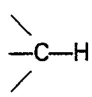

bonds, characterised by treating the perfluoropolyether in the liquid state, at a temperature from 150° to 200°C, with a gaseous reagent selected from oxygen, chlorine and fluorine, and in the presence of ultraviolet radiation, thus obtaining the conversion of the polluting substances into volatile degradation products, which are successively removed by degassing the perfluoropolyether.

2. A process as claimed in claim 1, characterized in that ultraviolet radiation of a wavelength from 240 to 600 nm is utilized.

3. A process as claimed in claim 1 or 2, characterized in that the degassing of the treated perfluoropolyether is effected under vacuum.

**Patentansprüche**

1. Verfahren zur Entfernung von organischen Verunreinigungen, die OH-Gruppen und/oder

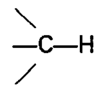

Bindungen enthalten, aus Perfluorpolyethern, dadurch gekennzeichnet, daß man den Perfluorpolyether in flüssiger Phase bei einer Temperatur von 150 bis 200°C in Anwesenheit ultravioletter Strahlung mit einem gasförmigen Reagens behandelt, welches ausgewählt ist aus Sauerstoff, Chlor und Fluor, wobei es zur Umwandlung der verunreinigenden Substanzen in flüchtige Abbauprodukte kommt, die durch Entgasung des Perfluorpolyethers sukzessiv entfernt werden.

2. Verfahren nach Anspruch 1, dadurch gekennzeichnet, daß ultraviolette Strahlung einer Wellenlänge von 240 bis 600 nm verwendet wird.

3. Verfahren nach Anspruch 1 oder 2, dadurch gekennzeichnet, daß die Entgasung der behandelten Perfluorpolyethers im Vakuum durchgeführt wird.

**Revendications**

1. Un procédé d'élimination dans des perfluoropolyéthers de substances polluantes organiques contenant des groupes —OH et des liaisons

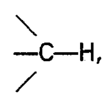

caractérisé en ce que l'on traite le perfluoropolyéther à l'état liquide à une température de 150 à 200°C, par un réactif gazeux choisi parmi: oxygène, chlore et fluor, et en présence de rayonnement ultraviolet, l'on obtient ainsi la conversion de ces substances polluantes en produits de dégradation volatiles que l'on élimine ultérieurement par dégazage du perfluoropolyéther.

2. Un procédé selon la revendication 1, caractérisé en ce que l'on utilise un rayonnement ultraviolet d'une longueur d'ondes comprise entre 240 et 600 nm.

3. Un procédé selon la revendication 1 ou 2, caractérisé en ce que le dégazage du perfluoropolyéther traité s'effectue sous vide.